# EUROPEAN PATENT APPLICATION

(11) **EP 3 006 555 A1**
(43) Date of publication of application: **13.04.2016**
(21) Application number: 14803347.5
(22) Date of filing: 30.05.2014
(51) Int. Cl.: C12N 1/20, A23C 9/123

(54) **FERMENTED MILK THAT DOES NOT UNDERGO INCREASE IN ACID LEVEL, AND METHOD FOR PRODUCING SAME**

(30) Priority: 31.05.2013 JP 2013116332
(71) Applicant: Meiji Co., Ltd., Tokyo 136-8908 (JP)
(72) Inventor: UCHIDA, Hideaki, Odawara-shi Kanagawa 250-0862 (JP); MURATA, Saori, Odawara-shi Kanagawa 250-0862 (JP); SAITOU, Mizue, Odawara-shi Kanagawa 250-0862 (JP); KIMURA, Katsunori, Odawara-shi Kanagawa 250-0862 (JP)
(74) Representative: TBK
(86) International application number: PCT/JP2014/064374
(87) International publication number: WO 2014/192905

(57) **Abstract**

The present invention relates to: fermented milk that does not undergo the increase in an acid level; a method for producing the fermented milk; *Lactobacillus delbrueckii* subsp. *bulgaricus* OLL1171 (NITE BP-01569) which is a lactic acid production strain useful for the production of fermented milk that does not undergo the increase in an acid level; and a method for screening for a lactic acid production strain useful for the production of fermented milk that does not undergo the increase in an acid level.

## Description

### [Technical Field]

The present invention relates to fermented milk wherein an increase in acidity is suppressed, and a production method thereof, as well as a lactic acid-producing bacterial strain useful for the production of fermented milk wherein an increase in acidity is suppressed, and a screening method therefor.

### [Background Art]

In many of the fermented milk produced using lactic acid-producing bacteria and even in their final products, live lactic acid-producing bacteria are contained. The final products are usually refrigerated in order to maintain flavor of the fermented milk. However, even during refrigerated storage, acidity increases with gradual progression of fermentation due to live lactic acid-producing bacteria, and therefore it is difficult to maintain constant quality of the fermented milk for a long period of time. Such phenomenon is referred to as post-acidification or after-acidification, and is distinguished from the acid generation that is indispensable for the production of fermented milk.

As a method for producing fermented milk wherein an increase acidity is suppressed during refrigerated storage, a method in which activity of lactic acid bacteria in the fermented milk is decreased by pressure treatment after fermentation has been proposed (Patent Document 1).

In addition, a method for suppressing an acidity increase during storage of the fermented milk by compounding chitosan has been proposed (Patent Document 2).

Furthermore, a method for producing fermented milk wherein an acidity increase during storage is suppressed by co-using an acid-production suppressing strain of *Lactobacillus bulgaricus* and a viscous-substance producing strain of *Streptococcus thermophilus* as the starter lactic acid bacteria, has been proposed (Patent Document 3). Moreover, a method for producing fermented milk wherein an acidity increase during storage is suppressed by using a low-temperature-sensitive strain of lactic acid bacteria *Streptococcus thermophilus,* has been proposed (Patent Document 4).

### [Prior Art Documents]

### [Patent Documents]

[Patent Document 1] JP A 04-075555
[Patent Document 2] JP A 03-292853
[Patent Document 3] JP A 07-236416
[Patent Document 4] JP A 2000-270844

### [Summary of the Invention]

### [Problems to Be Solved by the Invention]

However, according to studies of the present inventors, in the invention of Patent Document 1, high-pressure treatment of entire product after completion of fermentation is necessary, so that the method is not practical. In the invention of Patent Document 2, since the use of chitosan is essential, the method cannot be employed in the production of plain yogurt. Furthermore, in the invention of Patent Document 3, since the use of yeast extract, etc. is required to promote fermentation and to suppress delay of fermentation time, the acidity achieved by conventional fermentation time is also decreased. In the invention of Patent Document 4, fermented milk is produced using a lactic acid bacteria starter that has been prepared using yeast extract.

As described above, while various methods have been proposed in order to suppress post-acidification, a production method of fermented milk wherein an increase in acidity during refrigerated storage is suppressed, and wherein no additives to promote fermentation or to suppress acidity are required and no special production steps are required in usual fermentation time, has not yet been established. In addition, in conventional production methods of fermented milk wherein an increase in acidity during refrigerated storage is suppressed, in many cases fermentation time is long and preferred aroma of fermented milk is not rich.

Accordingly, an object of the present invention is to solve the problems of the prior art and to provide a fermented milk wherein an increase in acidity during refrigerated storage is suppressed, by means of usual production steps with a conventional level of fermentation time, without using additives to promote fermentation or to suppress acidity, as well as to provide a production method thereof.

Furthermore, the present invention also has an object to provide a lactic acid-producing strain which enables the production of fermented milk wherein an increase in acidity during refrigerated storage is suppressed, as well as a screening method therefor.

### [Means for Solving the Problems]

The present inventors have found that, during intensive study to solve the above problems, it is possible to suppress an increase in acidity during refrigerated storage without delaying the fermentation, by using an acid-sensitive lactic acid-producing strain to produce fermented milk; as a result of further investigation, the present inventors have completed the invention.

Namely, the present invention relates to the following.
[1] *Lactobacillus delbrueckii* subsp. *bulgaricus* OLL1171 (NITE BP-01569).
[2] A fermented milk comprising *Lactobacillus delbrueckii* subsp. *bulgaricus* OLL1171 (NITE BP-01569).
[3] The fermented milk according to [2], wherein pH is 4.1-4.7.
[4] The fermented milk according to [2] or [3], which is refrigerated.
[5] The fermented milk according to [4], wherein an increase in acidity is suppressed.
[6] The fermented milk according to any one of [2] to [5], wherein the acidity is 0.7-1.0.
[7] The fermented milk according to any one of [1] to [6], wherein yeast extract in an amount of more than 0.05 wt% is not added.
[8] A fermented milk comprising lactic acid-producing bacterium which is microbiologically characterized in that, in a skim milk medium wherein its pH is adjusted to 4.2-4.3 by the addition of lactic acid, to which the lactic acid-producing bacterium after activation is inoculated, and which is then maintained at 35-47°C, 15 h or longer time is required until the pH value is decreased by 0.2.
[9] The fermented milk according to [8], wherein the lactic acid-producing bacterium is *Lactobacillus delbrueckii* subsp. *bulgaricus* OLL1171 (NITE BP-01569).
[10] A method for producing fermented milk, comprising a step of adding *Lactobacillus delbrueckii* subsp. *bulgaricus* OLL1171 (NITE BP-01569) as a starter bacterium to a raw material mix.
[11] The production method according to [10], comprising a step of fermentation at 35-47°C for 3-4 h.
[12] The production method according to [10] or [11], wherein the fermentation step is terminated when the acidity reaches between 0.7 and 0.8, inclusive.
[13] The production method according to any one of [10] to [12], comprising a step of deoxygenation of the raw material mix.
[14] The production method according to any one of [10] to [13], wherein yeast extract in an amount of more than 0.05 wt% is not added to the starter.
[15] A method for producing fermented milk, comprising a step of adding lactic acid-producing bacterium as a starter bacterium to a raw material mix, wherein said lactic acid-producing bacterium is microbiologically characterized in that, in a skim milk medium wherein its pH is adjusted to 4.2-4.3 by the addition of lactic acid, to which the lactic acid-producing bacterium after activation is inoculated, and which is then maintained at 35-47°C, 15 h or longer time is required until the pH value is decreased by 0.2.
[16] A method for screening acid-sensitive lactic acid-producing bacteria, comprising: preparing a skim milk medium wherein its pH is adjusted to 4.2-4.3 by the addition of lactic acid, inoculating activated test bacteria to the medium and maintaining the medium at 35-47°C, and measuring changes in the pH value in the medium.

### [Advantageous Effects of the Invention]

In the present invention, by using acid-sensitive lactic acid-producing bacteria, in particular *Lactobacillus delbrueckii* subsp. *bulgaricus* OLL1171 strain (NITE BP-01569) (herein also referred to as "OLL1171 strain"), it becomes possible to provide a fermented milk wherein an increase in acidity during refrigerated storage is suppressed, without requiring the usage of additives to promote fermentation or to suppress an acidity increase, and without using special production steps, in a conventional level of fermentation time.

In particular, the present invention enables to suppress an increase in acidity and a decrease in pH value over time in the fermented milk during refrigerated storage, and enables to suppress changes in the flavor of the fermented milk during distribution after production or during storage compared to conventional products, while keeping refreshing flavor and the number of viable cells of lactic acid-producing bacteria for a long period of time.

Furthermore, the invention enables to screen for, within a short period of time, acid-sensitive lactic acid-producing bacterial strains useful for producing fermented milk with a suppressed increase in acidity.

### [Brief Description of Drawings]

[Fig. 1] Figure 1 is a diagram showing effects of pH value (pH: 4.5) on the fermentation by *L. bulgaricus* at 43°C.
[Fig. 2] Figure 2 is a diagram showing effects of pH value (pH: 4.25) on the fermentation by *L. bulgaricus* at 43°C.
[Fig. 3] Figure 3 is a diagram showing effects of pH value (pH: 4.0) on the fermentation by *L. bulgaricus* at 43°C.
[Fig. 4] Figure 4 is a diagram showing effects of pH value (pH: 4.5) on the storage of *L. bulgaricus* at 10°C.
[Fig. 5] Figure 5 is a diagram showing effects of pH value (pH: 4.25) on the storage of *L. bulgaricus* at 10°C.
[Fig. 6] Figure 6 is a diagram showing effects of pH value (pH: 4.0) on the storage of *L. bulgaricus* at 10°C.

### [Modes for Carrying out the Invention]

The "fermented milk" in the present specification refers to milk that has been fermented, and includes the "fermented milk" defined under the Ministerial Ordinance on Milk and Milk Products Concerning Compositional Standards, etc. (Ministerial Ordinance on Milk, etc.), "lactic acid bacteria beverage", "milk beverage", and "natural cheese" etc.. For example, fermented milk refers to the "fermented milk" defined under the Ministerial Ordinance on Milk, etc., that is, those obtained by fermenting milk such as raw milk, cow's milk, special milk, raw goat's milk, pasteurized goat's milk, raw sheep's milk, composition modified milk, low fat milk, skim milk or processed milk, or milk, etc. containing an equal or greater amount of milk solids-not-fat compared thereto, with lactic acid bacteria or yeasts, and then forming a solid (hard type), a paste (soft type) or a liquid (drink type), or frozen products thereof.

Typical examples of fermented milk include yogurt. In the international standard defined by Food and Agriculture Organization (FAO)/World Health Organization (WHO), the following definition is made: "a product called yogurt is made from dairy products such as milk and skim milk powder by lactic acid fermentation action of both *Streptococcus salivarius* subsp. *thermophilus* and *Lactobacillus delbrueckii* subsp. *bulgaricus,* and large amounts of these two types of bacteria are present in the final product." As used herein, "yogurt" is intended to include the yogurt defined above by FAO/WHO.

Since yogurt contains a large amount of live lactic acid-producing bacteria, by using acid-sensitive lactic acid-producing bacteria of the present invention in the production of yogurt, the effect of suppressing an acidity increase during refrigerated storage of the fermented milk is clearly observed. Accordingly, in the present invention, suitable fermented milk includes yogurt, and lactic acid bacteria beverages, milk beverages and cheeses (natural cheese, processed cheese) containing live bacteria. In particular, yogurt that is stored under refrigeration, such as plain yogurt, hard yogurt (set type yogurt), soft yogurt, and yogurt drinks are suitable as fermented milk.

Hereinafter, the present invention will be described in detail focusing mainly on yogurt as the fermented milk. However, the present invention is not limited to such embodiments, and intends to include embodiments such as lactic acid bacteria beverages, milk drinks and cheeses. As for yogurt and other embodiments, those skilled in the art will be able to appropriately make modifications based on the description herein.

As used herein, "raw material mix" refers to a liquid containing milk components such as raw milk, whole milk, skim milk and whey. Here, the raw milk refers to animal milk such as cow milk. The raw material mix may contain, in addition to milk components such as raw milk, whole milk, skim milk and whey, the processed products thereof (such as whole milk powder, concentrated whole milk, skim milk powder, concentrated skim milk, condensed milk, whey powder, butter milk, butter, cream, cheese, casein, whey protein concentrate (WPC), whey protein isolate (WPI), α-lactalbumin (α-La), β-lactoglobulin (β-Lg), etc.).

Incidentally, the raw material mix may contain, in addition to the milk components, soymilk, sugar, saccharides, sweeteners, flavors, fruit juices, fruit pulp, vitamins, minerals, food products such as oils and fats, food ingredients and food additives, etc. In addition, the raw material mix may contain, if necessary, stabilizers, thickeners, and gelling agents, etc. such as pectin, soy polysaccharides, CMC (carboxymethylcellulose), agar, gelatin, carrageenan, and gums.

In the present invention, preferably, the raw material mix only contains those which are commonly used in the production of fermented milk, such as the above-mentioned food products, food ingredients, food additives and stabilizers, thickeners, and gelling agents. In particular, from the viewpoint of texture and flavor of fermented milk, it is desirable not to add (blend) components having fermentation-promoting effect and components having acidity-increase inhibitory effect, such as "yeast extract" and "chitosan," in fermented milk or to add them in a trace amount that does not exhibit such effects even if they are added in fermented milk.

In a preferred embodiment of the present invention, in the raw material mix, the amount of yeast extract added is, for example 0.01 wt% or less, preferably 0.005 wt% or less, more preferably 0.001 wt% or less, furthermore preferably 0.0005 wt% or less, particularly preferably 0.0001% or less, and most preferably, no yeast extract is contained. Also in a preferred embodiment of the present invention, in the fermented milk, the amount of yeast extract added is, for example 0.01 wt% or less, preferably 0.005 wt% or less, more preferably 0.001 wt% or less, furthermore preferably 0.0005 wt% or less, particularly preferably 0.0001 wt% or less, and most preferably, no yeast extract is contained.

Furthermore, in a preferred embodiment of the present invention, in the raw material mix, the amount of chitosan added is, for example less than 0.01 wt%, preferably less than 0.005 wt%, more preferably less than 0.001 wt%, furthermore preferably less than 0.0005% wt%, particularly preferably less than 0.0001 wt%, and most preferably, no chitosan is contained. Also in a preferred embodiment of the present invention, in the fermented milk, the amount of chitosan added is, for example less than 0.01 wt%, preferably less than 0.005 wt%, more preferably less than 0.001 wt%, furthermore preferably less than 0.0005% by weight, particularly preferably less than 0.0001 wt%, and most preferably, no chitosan is contained.

As used herein, "comprising lactic acid-producing bacteria," and "comprising *Lactobacillus delbrueckii subsp bulgaricus* OLL1171 strain (accession number: NITE BP-01569)" means that fermented milk comprises said bacteria (said microorganisms), preferably comprises live cells of said bacteria. In addition to the cases wherein the obtained fermented milk comprises lactic acid-producing bacteria by using said bacteria as starter bacteria, it also includes cases wherein the fermented milk comprises lactic acid-producing bacteria by adding said bacteria before fermentation, during fermentation and after fermentation, in addition to lactic acid-producing bacteria used as the starter bacteria. As used herein, the term "starter bacteria" means microorganisms that ferment, and "starter" means a culture of starter bacteria.

As used herein, "acidity" means a measured value in accordance with "5. Method of measuring acidity of milk and dairy products" in Milk-related laws and regulations (Dairy Group Hygienic Association, March 2004), p. 56, and the details thereof are as follows. That is, in the present specification, "acidity" is a value of acidity measured as follows: "10 ml of a sample is diluted by adding the same amount of water without carbon dioxide gas, to which 0.5 ml of phenolphthalein solution as an indicator is added, and the sample is titrated using 0.1 mol/L sodium hydroxide solution for 30 s with a limit being set at the point where rose-pink color disappears; a percentage amount of lactic acid per 100 g of the sample is calculated from the obtained titer, and this value is determined to be the acidity. One ml of 0.1 mol/L sodium hydroxide solution corresponds to 9 mg of lactic acid. As the indicator, 1 g of phenolphthalein is dissolved in 50% ethanol to make an amount of 100 ml."

In general, when the fermented milk to be shipped as a final product is yogurt, acidity of the fresh product (at the end of fermentation) is approximately 0.6-0.8%. Since fermented milk typically may be refrigerated for about 1 to 2 weeks (7-14 days), when the fermented milk is actually consumed, the acidity of the refrigerated products (after refrigerated storage) can rise to approximately 0.9-1.2%. In this case, from the viewpoint of texture and flavor (in particular, sourness) of the fermented milk, the acidity of the fermented milk should be, for example, 0.6-1.1%, preferably 0.7-0.9%, and more preferably about 0.75-0.85%. In the present invention, since an increase in acidity during refrigerated storage of fermented milk is suppressed, the acidity of fresh products of fermented milk is, for example, 0.6-0.9%, preferably 0.65-0.85%, more preferably 0.7-0.8%; and after refrigerated storage for about 1-2 weeks, the acidity of the refrigerated products of fermented milk is, for example, 0.7-1.1%, preferably 0.75-1.0%, and more preferably 0.75-0.9%.

As used herein, "an increase in acidity is suppressed" means that an increase in the acidity from the end of fermentation to that after refrigerated storage for a certain period is 0.25% or less. In one embodiment of the present invention, a change (increase) in acidity after one week (7 days) of refrigerated storage (10°C) from the end of fermentation is, for example, 0.15% or less, preferably 0.13% or less, more preferably 0.11% or less, furthermore preferably 0.10% or less, and particularly preferably 0.09% or less. In this case, the lower limit value of a change (increase) in acidity is not particularly limited, and it is for example 0.02%.

In one embodiment of the present invention, a change (increase) in acidity after two weeks (14 days) of refrigerated storage (10°C) from the end of fermentation is, for example, 0.25% or less, preferably 0.23% or less, more preferably 0.21% or less, furthermore preferably 0.20% or less, and particularly preferably 0.19% or less. In this case, the lower limit value of a change (increase) in acidity is not particularly limited, and it is for example 0.05%.

In one embodiment of the present invention, the acidity at one week (7 days) of refrigerated storage (10°C) from the end of fermentation is, for example, 0.6-0.95 %, preferably 0.65-0.9%, more preferably 0.7-0.85%, furthermore preferably 0.75-0.85%, and particularly preferably 0.8-0.85%. In one embodiment of the present invention, the acidity at two weeks (14 days) of refrigerated storage (10°C) from the end of fermentation is, for example, 0.7-1.05 %, preferably 0.75-1.0%, more preferably 0.8-0.95%, furthermore preferably 0.85-0.95%, and particularly preferably 0.9-0.95%.

As used herein, "refrigerated storage" refers to storage at a low temperature of 10°C or less, and typically it refers to storage at 5-10°C. In general, when the fermented milk to be shipped as a final product is yogurt, pH of its fresh product (at the end of fermentation) is approximately 4.0-5.0. In this case, from the viewpoint of texture and flavor (in particular, sourness) of the fermented milk, pH of the fermented milk should be, for example, 4.0 or more, preferably 4.1 or more, and more preferably 4.2 or more. In the present invention, since an increase in acidity during refrigerated storage of fermented milk is suppressed, pH of fresh products of fermented milk is, for example, 4.0-5.0, preferably 4.1-4.7, and more preferably 4.2-4.6; after refrigerated storage for about 1-2 weeks, pH of the refrigerated products of fermented milk is, for example 4.0-5.0, preferably 4.1-4.7, and more preferably 4.2-4.6.

In one embodiment of the present invention, a change (decrease) in pH after one week (7 days) of refrigerated storage (10°C) from the end of fermentation is, for example, 0.35 or less, preferably 0.32 or less, more preferably 0.3 or less, furthermore preferably 0.27 or less, and particularly preferably 0.25 or less. The lower limit value of the change (decrease) in acidity is not particularly limited, and it is for example 0.02.

In one embodiment of the present invention, a change (decrease) in pH after two weeks (14 days) of refrigerated storage (10°C) from the end of fermentation is, for example, 0.5 or less, preferably 0.47 or less, more preferably 0.45 or less, furthermore preferably 0.42 or less, and particularly preferably 0.4 or less. In this case, the lower limit value of the change (decrease) in pH is not particularly limited, and it is for example 0.05.

In one embodiment of the present invention, pH of the fermented milk after one week (7 days) of refrigerated storage (10°C) from the end of fermentation is, for example, 4.0-5.0, preferably 4.1-4.8, more preferably 4.2-4.6, furthermore preferably 4.3-4.6, and particularly preferably 4.4-4.6. In one embodiment of the present invention, pH of the fermented milk after two weeks (14 days) of refrigerated storage (10°C) from the end of fermentation is, for example, 4. 0-5. 0, preferably 4.1-4.8, more preferably 4.2-4.6, furthermore preferably 4.3-4.6, and particularly preferably 4.4-4.6.

In one embodiment of the present invention, when changes in pH and changes in acidity over time are within the above ranges, quality of the fermented milk can be controlled (maintained) at predetermined quality (in particular, certain flavors) for a long period of time, and the expiration date of the fermented milk can be sufficiently prolonged.

As used herein, "lactic acid-producing bacteria" refer to microorganisms that produce lactic acid, and include lactic acid bacteria belonging to *Lactobacillus, Streptococcus, Lactococcus, Leuconostoc* and *Pediococcus,* as well as *Bifidobacterium.* Here, preferable lactic acid-producing bacteria are *Lactobacillus bulgaricus (Lactobacillus delbrueckii* subsp. *bulgaricus,* herein also referred to as "Bulgaria bacteria" and "*L*. *bulgaricus"),* and *Streptococcus thermophilus (Streptococcus salivarius* subsp. *thermophilus,* herein also referred to as "thermophilus bacteria" and "*S*. *thermophilus"*)*,* which are essential for the production of yogurt, and particularly preferable bacteria are Bulgaria bacteria (L. *bulgaricus)* that produce a lot of lactic acid in the production of yogurt.

*Lactobacillus delbrueckii* subsp. *bulgaricus* OLL1171 (Bulgaria (*L. bulgaricus*) OLL1171) of the present invention has been deposited at the Patent Microorganisms Depositary, National Institute of Technology and Evaluation (2-5-8, Kazusa Kamatari, Kisarazu City, Chiba, 292-0818 Japan) on March 13, 2013, with the accession number: NITE BP-01569; and is *Lactobacillus delbrueckii* subsp. *bulgaricus* having the following characteristics.

*L. bulgaricus* OLL1171 has the following scientific properties (morphological properties, characteristics on a medium, physiological properties, etc.).
(a) Morphological properties
   Colony characteristics on a medium (BL(+) Agar, Nissui): basically circular (somewhat polymorphism), white to gray color, smooth type (partly rough type), flat
(b) Physiological properties
   Bacteria form: bacilli, Gram stain: positive, Lactic acid fermentation type: homo-lactic acid fermentation, Aerobic growth: +
(c) Other properties that characterize the microorganism Because subspecies lower than subsp. cannot be distinguished by 16sRNA gene sequence, *groEL* sequence analysis can be utilized in the identification of 4 subspecies of *L. delbrueckii (delbrueckii, bulgaricus, lactis, indicus),* and those of the following sequence can be utilized in the identification of OLL1171.
*groEL* sequence (OLL1171 strain):

AAAGGCCACCAAAGCAGCCGTTGACCAATTGCACAAGAACAGCCACAAAGTTTCCAGCCGG
GACCAAATTGCCCAAGTTGCTTCAATCTCAAGTGCTTCAAAGGAAATCGGCGACTTGATCG
CTGAAGCCATGGAAAAGGTCGGCAAGGACGGTGTTATCACCATTGAAGACTCCCGCGGAT
CGAAACTGAACTGAGCGTGGTTGAAGGGATGCAATTCGACCGCGGCTACCTGTCCCAATAC
ATGGTAACGGACAACGACAAGATGGAAGCTGACTTGGAAAACCCATACATCTTGATCACTG
ACAAGAAGATTTCCAACATCCAGGACATCTTGCCAATGTTGCAGGAAATCGTGCAAC
(SEQ ID No. 1)

Furthermore, *L. bulgaricus* OLL1171 has a characteristic of suppressing an increase in acidity of fermented milk as shown in Example 1, in addition to the above properties.

In the method for producing fermented milk of the present invention, conventional production steps of fermented milk can be adopted, and their preferred embodiments are described below.

The method for producing fermented milk of the present invention comprises a blending step of raw material mix, in which raw materials are mixed (blended). In the blending step of raw material mix, conditions generally used in the production of fermented milk may be employed as appropriate. In addition, in the production method of fermented milk of the present invention, the following steps are comprised similar to conventional methods: a step of (heat) sterilization of raw material mix, a step of cooling raw material mix, a step of adding a starter, a fermentation step, and a step of cooling fermented milk; and it is desirable to include these steps in this order. In these steps, conditions generally used in the production of fermented milk may be employed as appropriate.

The method for producing fermented milk of the present invention may comprise a homogenization step of raw material mix. The method for producing fermented milk of the present invention may comprise the homogenization step of raw material mix, simultaneously with or subsequent to the blending step of raw material mix; prior to or subsequent to the sterilization step of raw material mix; simultaneously with, prior to or subsequent to the cooling step after the sterilization step of raw material mix; subsequent to the fermentation step; simultaneously with, prior to or subsequent to the cooling step after the fermentation step, in this order. The method for producing fermented milk of the present invention may comprise the homogenization step of raw material mix once or two or more times. In the homogenization step of raw material mix, when a homogenizer, etc. is used, the pressure of the homogenization is, for example 1-100 MPa, preferably 5-50 MPa, more preferably 8-30 MPa, and furthermore preferably 10-20 MPa in the treatment.

The method for producing fermented milk of the present invention may comprise a deoxygenation step to deoxygenize raw material mix. In the deoxygenation step, oxygen present in the raw material mix (dissolved oxygen concentration) is reduced or removed. As a method of reducing dissolved oxygen concentration (DO) in the raw material mix (deoxygenation method), for example, a gas replacement treatment with inert gas such as nitrogen gas, helium, neon, argon and xenon, a membrane separation treatment using oxygen permeable membrane, a degassing treatment at low pressure or in vacuum may be utilized. In the deoxygenation step, dissolved oxygen concentration of the raw material mix is reduced or removed to, for example, 5 ppm or less, preferably 3 ppm or less, more preferably 2 ppm or less, and furthermore preferably 1 ppm or less.

The method for producing fermented milk of the present invention may comprise a deoxygenation step, simultaneously with or subsequent to the blending step of raw material mix; subsequent to the homogenization step of raw material mix; prior to or subsequent to the sterilization step of raw material mix; simultaneously with, prior to or subsequent to the cooling step of raw material mix; simultaneously with, prior to or subsequent to the adding step of starter; simultaneously with, prior to or subsequent to the fermentation step; simultaneously with, prior to or subsequent to the cooling step of fermented milk, in this order. The method for producing fermented milk of the present invention may comprise the deoxygenation step once or two or more times.

In the deoxygenation step, fermentation time can be reduced by reducing the dissolved oxygen concentration of raw material mix at the start of the fermentation step. Furthermore, the effect of suppressing an acidity increase during refrigerated storage of fermented milk can be enhanced thereby. Thus, it is important that the dissolved oxygen concentration in the raw material mix is maintained at a reduced level at the start of the fermentation step, and therefore, the deoxygenation step is desirably carried out simultaneously with, immediately prior to or subsequent to the adding step of starter, or simultaneously with or immediately prior to the fermentation step.

The method for producing fermented milk of the present invention may comprise, as described above, a step of adding a starter. Starter may be a single starter wherein a single type of acid-sensitive lactic acid-producing bacteria are cultured, or a mixed starter wherein said bacteria are mixed with other microorganisms and cultured. In the present invention, microorganisms other than acid-sensitive lactic acid-producing bacteria can be used as further starter bacteria.

As components for culturing lactic acid-producing bacteria, the use of components contained in the raw material mix is preferable, and the use of only the components contained in the raw material mix is more preferable. In specific terms, the use of raw milk, whole milk, skim milk, whole milk powder, skim milk powder, concentrated whole milk, concentrated skim milk, whey, whey powder, buttermilk, butter, cream, cheese, casein, whey protein concentrate (WPC), whey protein isolate (WPI), α-lactalbumin (α-La) and β-lactoglobulin (β-Lg), etc. is preferable, and the use of raw milk, pasteurized milk, skim milk, skim milk powder, whole milk powder, concentrated whole milk, and/or concentrated skim milk is more preferable.

As a growth-promoting agent for culturing lactic acid-producing bacteria, extracts such as yeast extract and meat extract may be added to the medium for culturing starter bacteria. However, from the viewpoint of flavor of fermented milk, it is not desirable that such components are added to the raw material mix in a high concentration upon production of fermented milk. Therefore, the amount of growth-promoting agent contained in the starter is, for example, less than 0.1 wt%, preferably less than 0.05 wt%, more preferably less than 0.01 wt%, furthermore preferably less than 0.005 wt%, and particularly preferably less than 0.001 wt%; and most preferably, no growth-promoting agent is contained in the starter.

The number of lactic acid-producing bacteria in the starter is, for example, 10 -10¹³ cfu/mL, preferably 10⁶-10¹² cfu/mL, more preferably 10⁷-10¹¹ cfu/mL, and furthermore preferably 10⁸-10¹⁰ cfu/mL.

In the raw material mix, the amount of a starter added is set to be, for example, 1-10 wt%, preferably 1-8 wt%, more preferably 2-6 wt%, and furthermore preferably 2-4%. In the present invention, a starter can be added to the raw material mix in accordance with ordinary methods.

The method for producing fermented milk of the present invention comprises a fermentation step, as described above. In the present invention, from the viewpoint of efficiently obtaining fermented milk with good flavor and texture, the fermentation temperature is set to be, for example 30-50°C, preferably 35-47°C, more preferably 37-45°C, and furthermore preferably 40-43°C. In contrast, when the method comprises a deoxygenation step of raw material mix, from the viewpoint of efficiently obtaining fermented milk with good flavor and texture, it is desirable to carry out the fermentation at relatively low temperature; therefore, the fermentation temperature is set to be, for example 28-47°C, preferably 30-45°C, more preferably 32-43°C, and furthermore preferably 35-40°C.

In the present invention, from the viewpoint of efficiently obtaining fermented milk with good flavor and texture, the fermentation time is set to be, for example 1-48 h, preferably 2-24 h, more preferably 3-10 h, furthermore preferably 3-6 h, and particularly preferably 3-5 h. In contrast, when the method comprises a deoxygenation step of raw material mix, from the viewpoint of efficiently obtaining fermented milk with good flavor and texture, it is desirable to carry out the fermentation in relatively short time; therefore, the fermentation time is set to be, for example 1-36 h, preferably 1-12 h, more preferably 2-8 h, furthermore preferably 2-5 h, and particularly preferably 2-4 h.

In a fermentation step, from the viewpoint of obtaining fermented milk with good flavor and texture, fermentation is terminated when the acidity of the fermented milk reaches, for example 0.6-1.0%, preferably 0.65-0.95%, more preferably 0.7-0.9%, and furthermore preferably 0.75-0.85%. In a fermentation step, from the viewpoint of obtaining fermented milk with good flavor and texture, fermentation is terminated when pH of the fermented milk reaches, for example 4.15-4.75, preferably 4.2-4.7, more preferably 4.25-4.65, and furthermore preferably 4.3-4.6.

The method for producing fermented milk of the present invention may comprise a cooling step of fermented milk, as described above. In the cooling step of fermented milk, temperature of the fermented milk is decreased from fermentation temperature (e.g., 43°C) to a predetermined low temperature (e.g., 10°C). Then, in the cooling step of fermented milk, the temperature is decreased to 10°C or less with a cooling rate of fermented milk of, for example within 1-60 min, preferably 1-30 min, more preferably 1-10 min, and furthermore preferably 1-5 min. In this case, in order to maximally reduce acid generation in the cooling step of fermented milk, a faster cooling rate is desirable.

The method for producing fermented milk of the present invention may comprise a crushing step of the curd of fermented milk and/or a homogenization step of fermented milk. In the crushing step of the curd of fermented milk, for example, casein particles contained in the fermented milk (solid component of the curd) are finely dispersed in the whey by applying stirring force to the fermented milk, so that the structure of the fermented milk is microparticulated. In the homogenization step of fermented milk, for example, casein particles contained in the fermented milk (solid component of the curd) are finely dispersed in the whey by extruding the fermented milk through a narrow flow passage while applying pressure, so that the structure of the fermented milk is microparticulated.

The method for producing fermented milk of the present invention may comprise a crushing step of the curd of fermented milk and/or a homogenization step of fermented milk, subsequent to the fermentation step; simultaneously with, prior to or subsequent to the cooling step of fermented milk, in this order. In the crushing step of the curd of fermented milk, a tank with stirring blades or a static mixer can be used. In the homogenization step of fermented milk, when a homogenizer, etc. is used, the pressure of homogenization is, for example 1-100 MPa, preferably 5-50 MPa, more preferably 8-30 MPa, and furthermore preferably 10-20 MPa in the treatment. Here, the crushing step of the curd of fermented milk is used in soft type yogurt and drinking type yogurt, which are pre-fermentation-type fermented milk, and the homogenization step of fermented milk is used in drinking type yogurt, which is pre-fermentation-type fermented milk.

The method for producing fermented milk of the present invention may comprise a step of adding other components, wherein flavor substances, acidulants, nutrient enhancing substances, flavorings and stabilizers etc. are mixed with raw material mix and/or with fermented milk (mainly, fresh product). In the step of adding other components, fermented milk is mixed with a flowable thickened/liquid solution containing, for example, flavor substances (sugar, high-intensity sweeteners, sweeteners such as liquid sugar, pulp and juice of fruits and vegetables, etc., jams, sauces, preparations), acidulants (citric acid, lactic acid, etc.), nutrient enhancing substances (vitamins, minerals, insoluble salts (calcium phosphate, calcium carbonate, whey calcium, etc.)), flavorings (flavors), stabilizers (pectin, carboxymethylcellulose (CMC), carrageenan, soya polysaccharides, etc.).

In the case of pre-fermentation-type fermented milk (soft type yogurt and drinking type yogurt), the method for producing fermented milk of the present invention may comprise a step of adding other components, subsequent to the fermentation step; simultaneously with, prior to or subsequent to the cooling step of fermented milk; prior to or subsequent to the homogenization step, in this order. Furthermore, in the case of after-fermentation-type fermented milk (hard type yogurt and set type yogurt), the method for producing fermented milk of the present invention may comprise a step of adding other components, simultaneously with or subsequent to the blending step of raw material mix; prior to or subsequent to the sterilization step of raw material mix; simultaneously with, prior to or subsequent to the cooling step after the sterilization step of raw material mix, in this order. However, in the production steps of fermented milk, from the viewpoint of suppressing changes in temperature of other components, it is desirable that the adding step of other components is employed for pre-fermentation-type fermented milk.

In a preferred embodiment of the present invention, the additive amount of a stabilizer (pectin, carboxymethylcellulose (CMC), carrageenan, soya polysaccharides, etc.) in the raw material mix is, for example, 0.05-1.0 wt%, preferably 0.05-0.8 wt%, more preferably 0.1-0.5 wt%, furthermore preferably 0.1-0.4 wt%, particularly preferably 0.1-0.3 wt%; and most preferably, the raw material mix does not contain a stabilizer. In addition, in a preferred embodiment of the present invention, the additive amount of a stabilizer in the fermented milkis, for example, 0.05-1.0 wt%, preferably 0.05-0.8 wt%, more preferably 0.1-0.5 wt%, furthermore preferably 0.1-0.4 wt%, particularly preferably 0.1-0.3 wt%; and most preferably, the fermented milk does not contain a stabilizer.

The present invention also provides a method for selecting (screening) lactic acid-producing bacteria having acid sensitivity. As used herein, the term "acid-sensitive lactic acid-producing bacteria" refers to lactic acid-producing bacteria having a low activity under acidic conditions (especially at pH 4.5 or less). Specifically, acid-sensitive lactic acid-producing bacteria are lactic acid-producing bacteria that have low ability to produce lactic acid in fermented milk at pH value of 4.5 or less.

Such acid-sensitive lactic acid-producing bacteria can be screened by the following steps.
(1) Adding lactic acid to a skim milk medium (for example, it contains skimmed milk powder at 8-12 wt%), to prepare the skim milk medium in which pH is adjusted to 4.2-4.3.
(2) Obtaining test bacteria (microorganisms) activated using a skim milk medium containing yeast extract.
(3) Inoculating the test bacteria (above (2)) to the skim milk medium (above (1)), then measuring changes in pH in said skim milk medium while maintaining the temperature at 35-47°C.

More specifically, a skim milk medium with pH 4.2-4.3 can be prepared in the following manner. Namely, using skim milk powder, water, lactic acid or citric acid, and pectin, etc., a skim milk medium with pH 4.4-4.6 that is close to the pH value of fermented milk (fresh product) immediately after fermentation is prepared. Such a skim milk medium comprises milk solids-not-fat at, for example 8-12 wt%, preferably from 8.5-11.5 wt%, more preferably 9-11 wt%, furthermore preferably 9.5-10.5 wt%, and particularly preferably at 10 wt%. Also, such skim milk medium comprises a fat content of, for example 0.001-0.5 wt%, preferably 0.001-0.2 wt%, more preferably 0.001-0.1 wt%, furthermore preferably 0.001-0.05 wt%, and particularly preferably, it substantially comprises no fat. Here, to the skim milk medium in which pH is adjusted to approximately 4.4-4.6, lactic acid or citric acid is added to adjust the pH to 4.2-4.3, preferably about 4.25.

Test bacteria can be activated by culturing in a skim milk medium containing a growth-promoting agent. In this case, the skim milk medium can be prepared by using skim milk powder, water, yeast extract, meat extract, and chitosan, etc. Here, examples of growth-promoting agent may include yeast extract, meat extract, chitosan, etc. In a preferred embodiment of the present invention, the additive amount of a growth-promoting agent to the skim milk medium is, for example, 0.01-0.5 wt%, preferably 0.02-0.3 wt%, more preferably 0.03-0.2 wt%, furthermore preferably 0.05-0.15 wt%, and particularly preferably it is 0.1 wt%.

Using a skim milk medium containing a growth-promoting agent, test bacteria are cultured at a predetermined culture temperature for a predetermined culture time. In the present invention, from the viewpoint of efficiently obtaining test bacteria, the culture temperature is set to be, for example 30-50°C, preferably 32-45°C, more preferably 35-42°C, and furthermore preferably 37-40°C. Also in the present invention, from the viewpoint of efficiently obtaining test bacteria, the culture time is set to be, for example 2-48 h, preferably 6-36 h, more preferably 12-30 h, furthermore preferably 18-27 h, and particularly preferably it is 24 h. The test bacteria may be activated for once or two or more times.

After (heat) sterilization of the skim milk medium in which pH is adjusted to 4.2-4.3 in accordance with ordinary methods, test bacteria are inoculated and cultured in accordance with ordinary methods. In this case, it is preferable to measure the activity of the test bacteria using a skim milk medium in which pH is adjusted to a predetermined value, at normal fermentation temperature of the fermented milk.

In the screening method of the present invention, after inoculation of test bacteria using a skim milk medium in which pH is adjusted to a predetermined value, the test temperature is maintained at, for example 35-50°C, preferably 37-50°C, more preferably 40-47°C, furthermore preferably, 43-45°C, and particularly preferably it is maintained at 43°C. In the screening method of the present invention, after inoculation of test bacteria using a skim milk medium in which pH is adjusted to a predetermined value, the test time is set to be, for example 1-48 h, preferably 2-24 h, more preferably 3-10 h, furthermore preferably 3-6 h, and particularly preferably 3-5 h.

Changes in pH can be measured in accordance with ordinary methods. Specifically, it is preferable to measure changes over time using a pH meter (commercial product). Changes in pH are measured over time, for example for 3 h or more, preferably 5 h or more, more preferably 10 h or more, and furthermore preferably 24 h or more.

In one embodiment of the present invention, the method for screening method acid-sensitive lactic acid-producing bacteria comprises the following steps.
(1) Mixing skim milk powder, water, lactic acid, and pectin to prepare (blend) a skim milk medium with pH 4.5 (milk solids-not-fat: 9.5 wt%, fat content: 0.1 wt%), adding lactic acid (50 wt%) thereto to prepare skim milk media in which pH value is adjusted to 4.25 and 4.0.
(2) Obtaining test bacterium that is activated twice using a skim milk medium (milk solids-not-fat: 9.5 wt%, fat content: 0.1 wt%) containing 0.1 wt% of yeast extract.
(3) Inoculating the test bacterium (above (2)) to the skim milk medium (above (1)), then measuring changes in pH in said skim milk medium while maintaining the temperature at 43°C.

In the present invention, test bacteria showing small changes in pH when cultured in the above medium (lactic acid-producing bacteria which do not grow at low pH) can be certified as acid-sensitive lactic acid-producing bacteria. In one embodiment of the present invention, lactic acid-producing bacteria which require a certain time period for decreasing the pH value of the medium by 0.2 when the skim milk medium is maintained at 35-47°C, for example, a time period of 15 h or more, preferably 16 h or more, more preferably 17 h or more, and furthermore preferably 18 h or more, can be certified as acid-sensitive lactic acid-producing bacteria. In one embodiment of the present invention, lactic acid-producing bacteria which require a certain time period for decreasing the pH value of the medium by 0.2 when the skim milk medium is maintained at 40-45°C, for example, a time period of 15 h or more, preferably 16 h or more, more preferably 17 h or more, and furthermore preferably 18 h or more, can be certified as acid-sensitive lactic acid-producing bacteria.

In one embodiment of the present invention, lactic acid-producing bacteria which require a certain time period for decreasing the pH value of the medium by 0.1 when the skim milk medium is maintained at 35-47°C, for example, a time period of 7 h or more, preferably 8 h or more, more preferably 9 h or more, and furthermore preferably 10 h or more, can be certified as acid-sensitive lactic acid-producing bacteria. In addition, in one embodiment of the present invention, lactic acid-producing bacteria which require a certain time period for decreasing pH value of the medium by 0.1 when the skim milk medium is maintained at 40-45°C, for example, a time period of 7 h or more, preferably 8 h or more, more preferably 9 h or more, and furthermore preferably 10 h or more, can be certified as acid-sensitive lactic acid-producing bacteria.

In another embodiment of the present invention, lactic acid-producing bacteria which do not show a decrease in pH value of more than 0.25 when the skim milk medium is maintained at 35-47°C for 24 h, can be certified as acid-sensitive lactic acid-producing bacteria. In addition, in another embodiment of the present invention, lactic acid-producing bacteria which do not show a decrease in pH value of more than 0.25 when the skim milk medium is maintained at 40-45°C for 24 h, can be certified as acid-sensitive lactic acid-producing bacteria.

Hereinafter, the present invention will be further described in detail based on examples; however, the present invention is not limited to these examples, and various modifications are possible without departing from the technical idea of the present invention. In this specification, unless explicitly indicated, % means wt%.

### [Examples]

### [Example 1] Screening acid-sensitive lactic acid-producing bacteria

### *Preparation method of skim milk media (pH: 4.5, pH: 4.25, pH: 4.0)

Skim milk powder, water, lactic acid, and carbohydrate solution (pectin) were mixed to prepare a skim milk medium with pH 4.5 (milk solids-not-fat: 9.5 wt%, fat content: 0.1 wt%). After the skim milk medium was homogenized and stabilized with pectin, commercially available lactic acid (50 wt%, Wako Pure Chemical Industries, Ltd., Japan: special grade reagent) was added to prepare non-sterile media having pH 4.25 and pH 4.0. By preparing the media in this way, formation of lumps accompanying with the adjustment of pH in the media comprising milk components was suppressed. Each of these media was used for experiment after being heat-sterilized at 95°C for 5 min.

### *Preparation of test Lactic acid-producing bacteria

Lactic acid-producing bacteria tested:
Strain A: Lactobacillus delbrueckii subsp. bulgaricus used for Meiji Bulgaria Yogurt LB81 (plain type yogurt) (Meiji Co., Ltd. Japan: manufactured in April 2013)
Strain B: Lactobacillus delbrueckii subsp. bulgaricus used for Meij i Bulgaria Yogurt (fruit-filled soft type yogurt) (Meij i Co. , Ltd. Japan: manufactured in April 2013)
Strain C: *Lactobacillus delbrueckii* subsp. *bulgaricus* OLL1171 (NITE BP-01569)

Each type of the lactic acid-producing bacteria to be tested was activated twice using a skim milk medium (skim milk powder: 10 wt%, water: 90 wt%, yeast extract: 0.1 wt%), with a condition of 37°C for 24 h.

### *Study on acid sensitivity

As described above, the lactic acid-producing bacteria to be tested were activated (twice), and the skim milk media with pH of 4.5, 4.25 and 4.0 were inoculated with each of the culture solutions at 2 wt%, then electrodes of an automated monitoring pH meter for multiple samples (ABLE Corporation) were set and pH values were measured over time for approximately 1 day, using a constant temperature bath of 43°C.

### *Study on low-temperature sensitivity

As described above, strain A, strain B, and strains C (OLL1171 strain) were activated (twice), and the skim milk media with pH of 4.5, 4.25 and 4.0 were inoculated with each of the culture solutions at 10 wt%, then electrodes of an automated monitoring pH meter for multiple samples (ABLE Corporation) were set and pH values were measured over time for approximately 2 days, using a constant temperature bath of 10°C.

### *Results

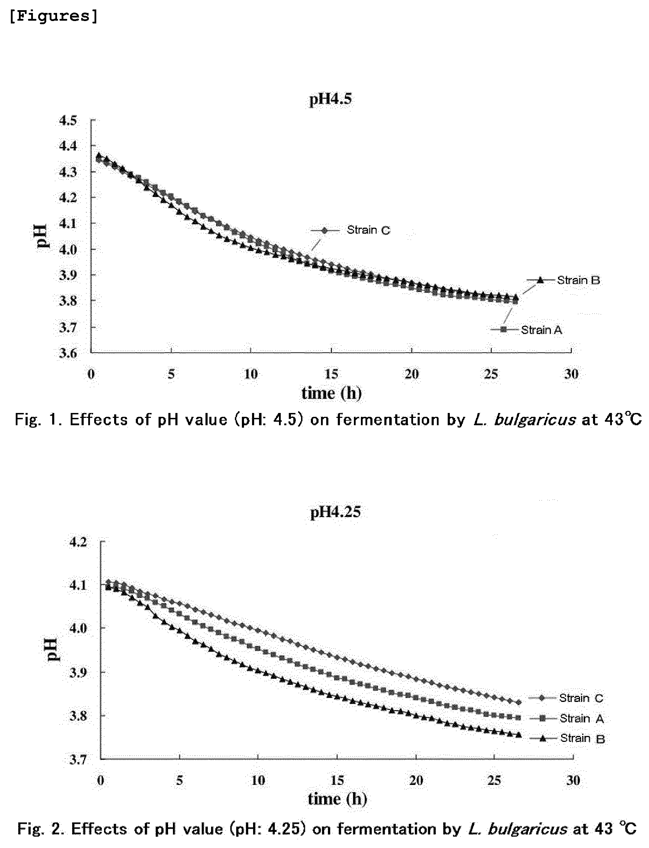

Results of measurement for the culture at 43°C are shown in Figs 1, 2 and 3.

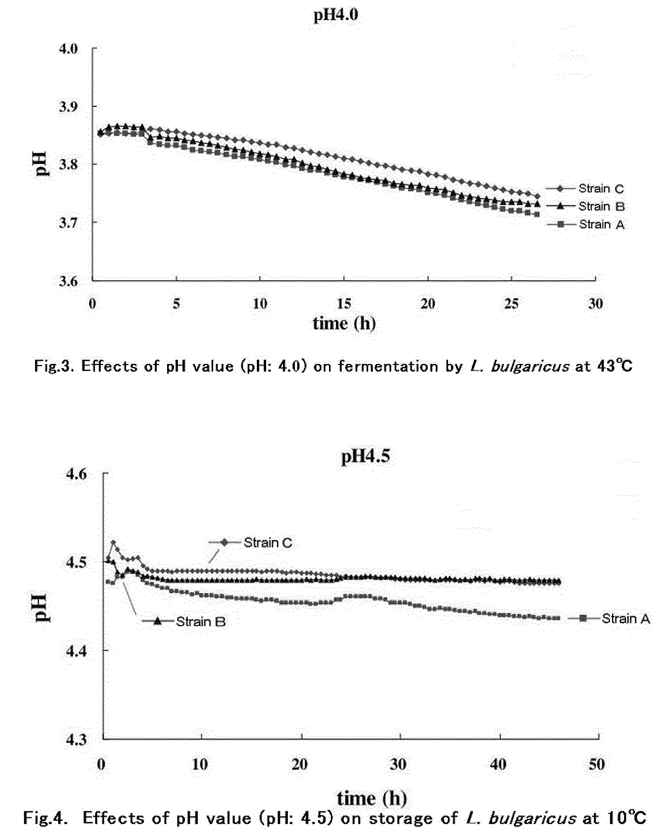

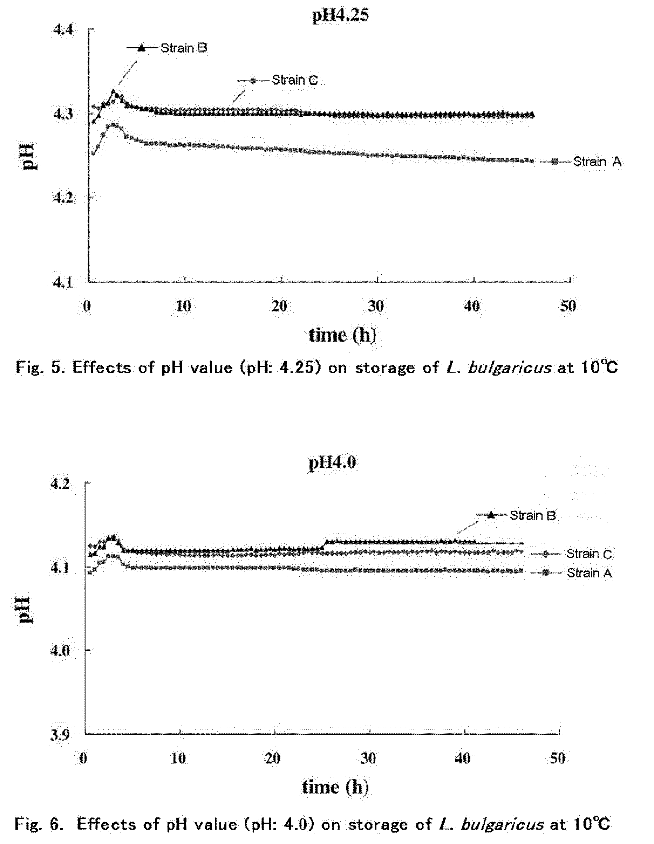

Results of measurement for the storage at 10°C are shown in Figs 4, 5 and 6.

### [Culture at 43°C] <pH: 4.5>

When culturing was carried out at 43°C using a skim milk medium with pH 4.5, which was close to the pH of fresh products of normal yogurt, three kinds of test bacteria showed good trends in growth curves (Fig. 1).

### [Culture at 43°C] <pH: 4.25>

When culturing was carried out at 43°C using a skim milk medium with pH 4.25, which was close to the pH of normal yogurt stored under refrigeration (10°C) for approximately 1-2 weeks, three kinds of test bacteria showed variations in growth curves (Fig. 2). The growth was good in the order of strain B > strain A > strain C (OLL1171 strain) . Regarding Δy/Δx (pH change/elapsed time), strain C = -0.0089, strain A = -0.0119, and strain B = -0.0130. Therefore, by the OLL1171 strain, a slight suppression of the growth due to lactic acid was indicated.

### [Culture at 43°C] <pH: 4.0>

When culturing was carried out at 43°C using a skim milk medium with pH 4.0, which was close to the pH of normal yogurt stored under refrigeration (10°C) for approximately 2-3 weeks, the growth of all the three kinds of test bacteria was slowed down. Regarding Δy/Δx (pH change/elapsed time), strain C = -0.00384 (Fig. 3). Because initial pH is identical, we can judge that strain A (*L. bulgaricus* for Meiji Bulgaria Yogurt LB81) showed the largest decrease in pH value.

### [Storage at 10°C]

In the storage at 10°C, a change in pH from the initial value was not observed, or only a slight change was observed in each *L. bulgaricus,* compared to the case of culturing at 43°C (Figs. 4, 5 and 6).

A slight change was observed only in the case of strain A using a skim milk medium of pH 4.5 and pH 4.25, and values of Δy/Ax (pH change/elapsed time) were strain A (pH: 4.5) = -0.000901, and strain A (pH: 4.25) = -0.000220.

Evaluation of acid sensitivity (pH: 4.25) and low-temperature sensitivity (10°C) of each *L. bulgaricus* is shown in Table 1 below.

[Table 1]

**Table 1.**

| | (lactic) acid sensitivity (pH: 4.25) | Low-temperature sensitivity (10°C) |
|---|---|---|
| Strain A | ++ | ++ |
| Strain B | + | +++ |
| Strain C (OLL1171 strain) | +++ | +++ |

### [Example 2] Production of fermented milk (yogurt)

A mixed starter in which *L. bulgaricus* used in Example 1 is combined with *S. thermophilus* was cultured using a skim milk medium (skim milk powder: 10 wt%, water: 90 wt%, sugar: 2 wt%). Raw materials of fermented milk (skim milk powder: 12.4 wt%, unsalted butter: 0.4 wt%, sugar: 5.4 wt%, water: 81.84 wt%) were mixed to prepare a raw material mix. The obtained raw material mix was heat-pasteurized at 95°C for 2 min, and cooled to 45°C. To the obtained raw material mix, the test starter was inoculated at 2 wt% and fermented at 43°C. When the acidity reaches 0.73%, fermentation was terminated and the sample was allowed to cool to 10°C, to obtain fermented milk. Then, the fermented milk was stored under refrigeration (10°C). Here, acidity and pH were measured in accordance with ordinary methods, and analytical results on day 1, day 7, and day 10 were shown in Table 2.

[Table 2]

**Table 2. Fermentability and refrigerated storage stability of fermented milk (10°C).**

| Kind of starter | Fermentation time | Day 1 (fresh product) | | Day 7 | | Day 14 | |
|---|---|---|---|---|---|---|---|
| | | Acidity [%] | pH [-] | Acidity [%] | pH [-] | Acidity [%] | pH [-] |
| Starter for Meiji Bulgaria Yogurt LB81 (*L. bulgaricus* strain A + *S. thermophilus* strain X) | 3 h | 0.83 | 4.50 | 1.04 | 4.11 | 1.09 | 4.05 |
| Starter for Meiji Bulgaria Fruit Yogurt (*L. bulgaricus* strain B + *S. thermophilus* strain Y) | 3 h 15 min | 0.75 | 4.65 | 0.90 | 4.32 | 1.04 | 4.10 |
| OLL1171 strain + *S. thermophilus* strain Y | 3 h 15 min | 0.76 | 4.57 | 0.84 | 4.36 | 0.93 | 4.22 |

From the results of Examples 1 and 2, it is understood that fermented milk showing a small increase in acidity during refrigerated storage can be obtained by using highly acid-sensitive (pH: 4.25) lactic acid-producing bacteria (L. *bulgaricus*)*.*

### [Example 3] Comparison of starter ability

In the same manner as in Example 2, fermented milk was obtained using commercially-available mixed starter with low post-acidification, YO-MIX 863 (DANISCO, Inc.). In addition, using a mixed starter in which OLL1171 strain are combined with *S. thermophilus* strain Y, three types of fermented milk were obtained under various condition. The obtained fermented milk was stored under refrigeration (10°C), and analyses were carried out on day 1, day 7, and day 10. Here, in the deoxygenation step, the raw material mix was subjected to nitrogen replacement after pasteurization until oxygen (dissolved oxygen concentration) reached 5 ppm or less. Acidity and pH were measured in accordance with ordinary methods, and analytical results on day 1, day 7, and day 10 were shown in Table 2. In addition, the number of live bacteria of Bulgaria bacteria (*L. bulgaricus:* LB) and thermophilus bacteria (*S. thermophilus:* ST) was measured in accordance with ordinary methods, and analytical results on day 1, day 7, and day 10 were shown in Table 2.

[Table 3]

**Table 3. Fermentability and refrigerated storage stability of fermented milk (10°C).**

| Kind of starter | Fermentation time | Day 1 (fresh product) | | Day 7 | | Day 14 | |
|---|---|---|---|---|---|---|---|
| | | Acidity [%] | pH [-] | Acidity [%] | pH [-] | Acidity [%] | pH [-] |
| OLL1171 strain + *S. thermophilus* strain Y Fermentation temperature: 43°C | 3 h 15 min | 0.76 | 4.57 | 0.84 | 4.36 | 0.93 | 4.22 |
| OLL1171 strain + *S. thermophilus* strain Y Fermentation temperature: 45°C | 3 h 15 min | 0.75 | 4.59 | 0.85 | 4.34 | 0.96 | 4.18 |
| OLL1171 strain + *S. thermophilus* strain Y Fermentation temperature: 45°C, with deoxygenation treatment | 3 h 10 min | 0.75 | 4.57 | 0.84 | 4.37 | 0.93 | 4.25 |
| YO-MIX863 Fermentation temperature: 43°C | 4 h 10 min | 0.79 | 4.43 | 0.86 | 4.33 | 0.91 | 4.24 |

From the results of Example 3 (Table 3) , when using the mixed starter in which OLL1171 strain is combined with *S. thermophilus* strain Y (mixed starter of the present invention), a shorter fermentation time can be set compared to the commercially available mixed starter (conventional mixed starter, prior art) ; accordingly, it is understood that the mixed starter of the present invention is a starter with high fermentability. Moreover, when using OLL1171 strain which is a highly acid-sensitive lactic acid-producing bacterium, it is understood that by carrying out a deoxygenation step, even shorter fermentation time can be set and an increase in acidity in the fermentation can be further suppressed.
[Table 4]

**Table 4. Number of live bacteria in refrigerated storage (10°C) [x10⁷ cfu/g].**

| Kind of starter | Day 1 (fresh product) [x10⁷ cfu/g] | | Day 7 [x10⁷ cfu/g] | | Day 14 [x10⁷ cfu/g] | |
|---|---|---|---|---|---|---|
| | LB | ST | LB | ST | LB | ST |
| OLL1171 strain + *S. thermophilus* strain Y Fermentation temperature: 43°C | 1.0 | 15.2 | 1.5 | 11.4 | 0.8 | 11.7 |
| OLL1171 strain + *S. thermophilus* strain Y Fermentation temperature: 45°C | 0.7 | 15.2 | 0.4 | 9.0 | 0.6 | 12.7 |
| OLL1171 strain + *S. thermophilus* strain Y Fermentation temperature: 45°C, with deoxygenation treatment | 0.4 | 17.7 | 0.8 | 11.1 | 0.3 | 15.0 |
| YO-MIX863 Fermentation temperature: 43°C | <0.1 | 76.0 | <0.1 | 87.0 | <0.1 | 51.0 |

| | | | | | | |
|---|---|---|---|---|---|---|
| LB: *L. bulgaricus,* ST: *S. thermophilus* (n=2, average) | | | | | | |

From the results of Example 3 (Table 4), when using the mixed starter in which OLL1171 strain is combined with *S. thermophilus* strain Y (mixed starter of the present invention), a large number of *Lactobacillus bulgaricus* was confirmed to be alive compared to the commercially available mixed starter (conventional mixed starter, prior art). Here, the following can be understood: in the case of mixed starter of the present invention, while a large number of live *Lactobacillus bulgaricus* is present during refrigerated storage, an increase in acidity in the fermented milk is suppressed based on the ability (characteristic) of the OLL1171 strain; on the other hand, in the case of the conventional mixed starter, an increase in acidity is suppressed due to extinction of *Lactobacillus bulgaricus* during refrigerated storage. That is, we can see that a large number of highly acid-sensitive lactic acid-producing bacteria, i.e., OLL1171 strain, is present as viable bacteria even during refrigerated storage.

### [Example 4] Evaluation of flavor of fermented milk (yogurt)

In the same manner as in Example 2, two kinds of mixed starter ([1] OLL1171 strain + *S. thermophilus* strain Y, [2] *L. bulgaricus* strain A + *S. thermophilus* strain X (starter for Meiji Bulgaria Yogurt LB81)) were used to prepare (produce) fermented milk (yogurt). Then, aroma components of these types of fermented milk were measured by headspace-GCMS (n=2).

Acetaldehyde is considered to be a basic compound which generates good flavor of yogurt, and is produced by *Lactobacillus bulgaricus.* Here, a measured value of acetaldehyde immediately after inoculation of *S. thermophilus* strain Y (single strain) was set to be 1.0 as a reference value. Then, relative values of acetaldehyde contained in the fermented milk prepared using the above two kinds of mixed starter were obtained.

Meiji Bulgaria Yogurt LB81 is plain type fermented milk (yogurt) having rich aroma. The relative value of acetaldehyde contained in this fermented milk is approximately 3-4 immediately after production (fresh product) as well as after 7 days of refrigerated storage. Meanwhile, the relative value of acetaldehyde contained in the fermented milk that was prepared using the mixed starter in which OLL1171 strain, which is a highly acid-sensitive lactic acid-producing bacterium, is combined with *S. thermophilus* strain Y, is 3 or more immediately after production (fresh product) as well as after 7 days of refrigerated storage, confirming that this fermented milk has a good flavor as yogurt.

## Claims

1. *Lactobacillus delbrueckii* subsp. *bulgaricus* OLL1171 (NITE BP-01569).

2. A fermented milk comprising *Lactobacillus delbrueckii* subsp. *bulgaricus* OLL1171 (NITE BP-01569).

3. The fermented milk according to Claim 2, wherein pH is 4.1-4.7.

4. The fermented milk according to Claim 2 or 3, which is refrigerated.

5. The fermented milk according to Claim 4, wherein an increase in acidity is suppressed.

6. The fermented milk according to any one of Claims 2 to 5, wherein the acidity is 0.7-1.0.

7. The fermented milk according to any one of Claims 1 to 6, wherein yeast extract in an amount of more than 0.05 wt% is not added.

8. A fermented milk comprising lactic acid-producing bacterium which is microbiologically **characterized in that**, in a skim milk medium wherein its pH is adjusted to 4.2-4.3 by the addition of lactic acid, to which the lactic acid-producing bacterium after activation is inoculated, and which is then maintained at 35-47°C, 15 h or longer time is required for until the pH value is decreased by 0.2.

9. The fermented milk according to Claim 8, wherein the lactic acid-producing bacterium is *Lactobacillus delbrueckii* subsp. *bulgaricus* OLL1171 (NITE BP-01569).

10. A method for producing fermented milk, comprising a step of adding *Lactobacillus delbrueckii* subsp. *bulgaricus* OLL1171 (NITE BP-01569) as a starter bacterium to a raw material mix.

11. The production method according to Claim 10, comprising a step of fermentation at 35-47°C for 3-4 h.

12. The production method according to Claim 10 or 11, wherein the fermentation step is terminated when the acidity reaches between 0.7 and 0.8, inclusive.

13. The production method according to any one of Claims 10 to 12, comprising a step of deoxygenation of the raw material mix.

14. The production method according to any one of Claims 10 to 13, wherein yeast extract in an amount of more than 0.05 wt% is not added to the starter.

15. A method for producing fermented milk, comprising a step of adding lactic acid-producing bacterium as starter bacterium to a raw material mix, wherein said lactic acid-producing bacterium is microbiologically **characterized in that**, in a skim milk medium wherein its pH is adjusted to 4.2-4.3 by the addition of lactic acid, to which the lactic acid-producing bacterium after activation is inoculated, and which is then maintained at 35-47°C, 15 h or longer time is required until the pH value is decreased by 0.2.

16. A method for screening an acid-sensitive lactic acid-producing bacterium, comprising: preparing a skim milk medium wherein its pH is adjusted to 4.2-4.3 by the addition of lactic acid, inoculating activated test bacterium to the medium and maintaining the medium at 35-47°C, and measuring changes in the pH value in the medium.


SEQUENCE LISTING

<110>  Meiji Co., Ltd.

<120>  Fermented milk with inhibited acidification and method for
       producing the same

<130>  PCT2646ME

<160>  1

<170>  PatentIn version 3.5

<210>  1
<211>  362
<212>  DNA
<213>  Lactobacillus bulgaricus

<400>  1
aaaggccacc aaagcagccg ttgaccaatt gcacaagaac agccacaaag tttccagccg      60

ggaccaaatt gcccaagttg cttcaatctc aagtgcttca aaggaaatcg gcgacttgat     120

cgctgaagcc atggaaaagg tcggcaagga cggtgttatc accattgaag actcccgcgg     180

gatcgaaact gaactgagcg tggttgaagg gatgcaattc gaccgcggct acctgtccca     240

atacatggta acggacaacg acaagatgga agctgacttg gaaaacccat acatcttgat     300

cactgacaag aagatttcca acatccagga catcttgcca atgttgcagg aaatcgtgca     360

ac                                                                    362